# EUROPEAN PATENT APPLICATION

(11) **EP 0 666 065 A1**
(43) Date of publication of application: **09.08.1995**
(21) Application number: 94300790.6
(22) Date of filing: 02.02.1994
(51) Int. Cl.: A61F 2/06

(54) **Stent for biliary, urinary or vascular system**

(71) Applicant: Mori, Katsushi, Toyonaka-shi, Osaka (JP)
(72) Inventor: Mori, Katsushi, Toyonaka-shi, Osaka (JP)
(74) Representative: Madeley, June Margaret

(57) **Abstract**

A stent is made of shape memory alloy which has an Austenite finished temperature (Af) ranging from 41°C to 43°C. The stent is capable of adopting a first configuration at a temperature below the Af and a second expanded configuration at or above the Af. In its first configuration the stent comprises a distal cylindrical portion (2), a proximal cylindrical portion (1), the two portions (1, 2) being connected with an interspace (3) between them. At the Af temperature the stent is reoriented, and at and above the Af temperature the proximal portion (1) flares into a funnel conical shape, expanding in maximum diameter and increasing in diameter away from the distal portion. The distal cylindrical portion (2) also expands in diameter, and the two portions directly connect with each other without the interspace (3).

## Description

This invention relates to a stent suitable for use with a biliary, urinary or vascular system. The invention concerns in particular a stent of shape memory alloy (SMA) to be placed in a pathologic stricture portion of the biliary, urinary or vascular system in the human body.

Urethral stents made of a shape memory alloy have been developed and tested in small clinical trials. However such prior art stents have failed to provide a useful, practical treatment of prostatic obstruction.

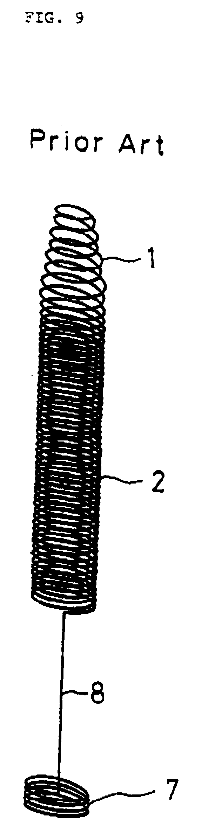

An example of a prior art stent is shown in Fig. 9. This comprises a spiral coil 2 having a tapered proximal end 1 and a hinge 7 at its distal end to aid removal of the stent. (In this context, that end of the stent which is to extend furthest into the patient's body is referred to as the "proximal" end of the stent.) Another type of stent, which is not illustrated, is a cross woven cylindrical stent, which would have a diameter of 12mm, for example.

Such prior art stents would have Austenite finished temperatures (Af) of about 48°C or 36°C, respectively. Thus, the stents would transform in shape at their Af.

In this context it is noted that the definition of Af is well known in the field of metallurgy, as are techniques for measuring it. It will be appreciated that an SMA transforms in shape at and around the Af temperature. Once it has transformed its shape, at and above the Af temperature it maintains the transformed shape. An SMA does not revert to the original shape until the temperature drops down to the Martensitic (Mf) temperature for the SMA.

We have found that problems arise with such prior art stents. An SMA stent having an Af of 48°C must be specially designed so that it does not burn the mucosa of the human body. In fact, the mucosa of the human body will be burned at temperatures of about 43°C or above.

In addition, we have found that an SMA stent having an Af of 36°C is liable to change its shape inadvertently. For example, it could change its shape during transportation or storage, since it would not be unusual for the temperature to rise above 36°C. In addition, the stent could perhaps be subjected to temperatures greater than 36°C in a doctor's or nurse's hand, and thus transforms its shape as it is being positioned in a patient's body and before it is in the desired location.

Furthermore, mesh stents require the assistance of an endoscope or other special accessory tool under ultrasonic or fluoroscopic control for accurate positioning and removal.

A stent may have to be removed for a variety of reasons, such as for example, once it has performed its function and is no longer necessary, or perhaps if it becomes encrusted, or alternatively if it migrates from its desired location such as into a bladder cavity. Thus, it can be very time consuming to use such stents. Also, they may occasionally traumatise the urethra, a blood vessel or a bile duct, which in turn may lead to bleeding or intolerable pain for patients.

The present invention aims to alleviate at least some of the aforementioned problems associated with prior art stents.

The present invention seeks to provide a stent which may be used in a less invasive manner, thus preferably requiring no sedation in the treatment of infravesical obstruction or the like.

The present invention also aims to provide a stent which is readily locatable in a desired position and removable from a patient simply on an out-patient basis.

Accordingly, in one broad aspect the present invention provides a stent made of shape memory alloy which has an Austenite finished temperature (referred in this text to as Af) ranging from about 41°C to about 43°C, the stent having a first configuration at a temperature below the Af and a second expanded configuration at or above the Af.

Thus, in embodiments of the present invention, the stent is selected to have an Af temperature of from 41°C to 43°C so that the mucosa of a patient is not burned when the stent is used. Furthermore, the present stents are less likely to transform in shape inadvertently during transportation and storage or even when handled by a doctor, than the prior art stents.

In embodiments of the invention the stent preferably comprises, in its first configuration, a distal cylindrical portion, a proximal cylindrical portion and an interspace therebetween. The stent adopts this first configuration at a temperature below the Af temperature. However, at the Af temperature the stent is reoriented to adopt a second, expanded configuration.

Thus, at and above the Af temperature, in the preferred embodiment the proximal portion preferably flares into a funnel shape which increases in diameter in the direction away from the distal portion. The distal cylindrical portion also expands in diameter, in comparison with its first configuration and the two portions directly connect with each other without the interspace. In particularly preferred embodiments the distal and proximal portion are linked by a straight wire portion in the first configuration.

In some embodiments the proximal portion and/or the distal portion of a stent are coiled. In other embodiments the proximal portion and/or the distal portion are made of mesh.

In preferred embodiments, when the stent is in its first configuration, the proximal and distal portions of the stent are of ellipsoidal or circular cross section.

Preferably when a stent is in its first configuration the distal portion is of longer length than the proximal portion.

It is also preferred to include means for assisting withdrawal of a stent from a patient. For example, in preferred embodiments the stent includes a cord or similar at or near the distal end which may be pulled by a doctor to remove the stent. Alternatively, the distal end of the stent may be made of a material which is attractive to a magnet and thus the stent may be drawn out of the body with the aid of a magnet.

In another broad aspect the present invention concerns a method of locating a stent made of a shape memory alloy having an Af ranging from about 41°C to about 43°C in a desired position in a biliary, urinary or vascular system. In this aspect, the method comprises inserting a stent in a first configuration into such a system and subsequently raising the temperature of the stent to or above its Af so that the stent assumes a second configuration.

In preferred embodiments of this aspect of the invention the temperature of the stent is raised by contacting the stent with fluid having a temperature at or above the Af of the stent. Preferably the stent is mounted on a container for fluid, such as a catheter, and the catheter is subsequently filled with fluid of the desired temperature to cause the stent to adapt its second configuration.

Particularly preferred embodiments involve the use of a stent which has, in its first configuration, a proximal cylindrical portion, a distal cylindrical portion and an interspace therebetween. The stent is preferably mounted on an inflatable catheter. The catheter and stent are inserted into the system and the catheter is inflated at least at or near the region of the interspace. In this way the catheter may bulge outwardly through the interspace. The catheter may be inflated by filling it with fluid at a temperature below the Af of the stent. The bulged or inflated part of the catheter may be used to assist accurate positioning of the stent. Once the stent is in a desired position the catheter is preferably drained and subsequently flushed with fluid at a temperature at or above the Af, which causes the stent to adopt its second configuration.

A stent of the present invention may thus be placed in position as follows in a particularly preferred embodiment of the present invention.

The stent is located around a catheter, and the catheter and stent are inserted into a biliary or urinary tract or a blood vessel in such a way that the proximal portion and the interspace portion pass through the stricture part of the tract/vessel. 2 to 5 ml of fluid such as water is passed into the catheter, causing the bag portion of the catheter to bulge or expand. The catheter with the stent located on it is then moved until the bulged bag portion abuts against the narrowed wall in the tract or blood vessel. The catheter is deflated and subsequently a flush of fluid such as saline at the Af temperature or above is fed into the catheter to reorient the stent so that it adopts its expanded configuration. The catheter is then withdrawn from the expanded stent leaving the stent in situ.

In embodiments of the present invention, when the stent is in an expanded configuration at and above the Af temperature, the neck of the flared proximal portion fits within a narrowing part of the stricture, so that the stent is located firmly in position. Also, when locating the stent in the desired position, the catheter is readily removed from the stent because the stent has expanded in diameter.

Embodiments of the present invention will now be described further, by way of example, with reference to the accompanying drawings in which:
Fig. 1 is a perspective view of an example SMA urethral stent in its cold phase (Martensitic phase) configuration;
Fig. 2 is a perspective view of a hot phase (Af) configuration of the SMA urethral stent of Fig. 1;
Fig. 3 is an explanatory diagram showing the example SMA stent fitted around a catheter;
Fig. 4. is an explanatory diagram showing a Foley catheter carrying an example spiral SMA urethral stent which is passed over a narrow urethra into a bladder cavity.
Fig. 5 is an explanatory diagram showing how the SMA urethral stent of Fig. 4 is accurately positioned by inflating the catheter bag;
Fig. 6 is an explanatory diagram showing the SMA urethral stent of Fig. 5 being left in situ at a desired position in the urethra at the prostate.
Fig. 7 is an explanatory diagram showing how the SMA urethral stent of Fig. 6 is removed by cooling;
Fig. 8 is an explanatory diagram showing how an example SMA stent is accurately positioned in a bilinary tract or a blood vessel by inflating a catheter bag;
Fig. 9 is a perspective view of a conventional urethral stent.

### EXAMPLES

A urethral stent according to embodiments of the present invention is capable of adopting different configurations depending upon its temperature. An example stent made of titanium and nickel, so called Shape Memory Alloy (SMA). The transforming temperature of this SMA is in the range of from 41°C to 43°. In this text this temperature is referred to as Af (Austenite finished).

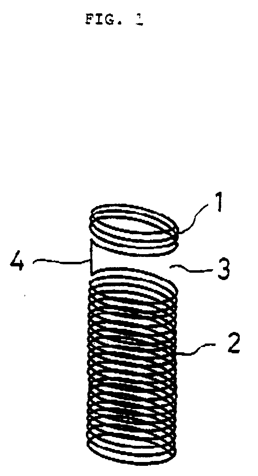

As shown in Fig. 1, at an ambient temperature below Af, the configuration of the example SMA stent is substantially cylindrical. It includes a distal coil portion 2, a proximal coil portion 1 and a straight wire portion 4. In this state both the coil portions 1, 2 are tightly coiled and the length of the proximal coil portion 1 is short, while that of the distal coil portion 2 is relatively long. There is an interspace 3 between the two coil portions 1, 2 and the straight wire portion 4 connects the two coil portions 1, 2. The cylindrical coil portions may be ellipsoidal or circular in cross section.

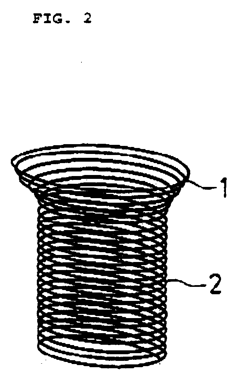

At an ambient temperature of and above the Af, as shown in Fig. 2, the SMA stent is reoriented to a wholly expanded coil with an anchoring conical portion 1 tapering in diameter and a long circular cylindrical portion 2. The wire portion 4 is absorbed into the shape combined the portions 1, 2. Thus, there is no longer an interspace 3 between the two portions 1, 2.

In an example SMA urethral stent, at the Martensitic state or below the Af temperature, the proximal coil portion 1 is, for example, about 5mm both in length and diameter, the distal coil portion 2 is about 45mm in length and about 5mm in diameter, and the distance of the interspace 3 is about 10mm. At the Af temperature, each respective portion of the SMA urethral stent expands in width. In this embodiment, the proximal coil portion 1 becomes 3mm in length and the flared end thereof measures 15mm in diameter. The distal portion 2 becomes 30mm in length and 10mm in diameter. The entire length of the stent is to be maintained at 10mm or more in diameter to secure the urinary bladder empty without any encrustation or impact, so that the stent may be retained for a longer period.

It will be appreciated that the present invention is not limited to a coiled stent, but also may be applied to a mesh stent or the like. Also other shape memory alloys with boundary temperature ranging from about 41°C to about 43°C, such as of copper and zinc, may be utilized.

A method for insertion and removal of such an SMA urethral stent is described below.

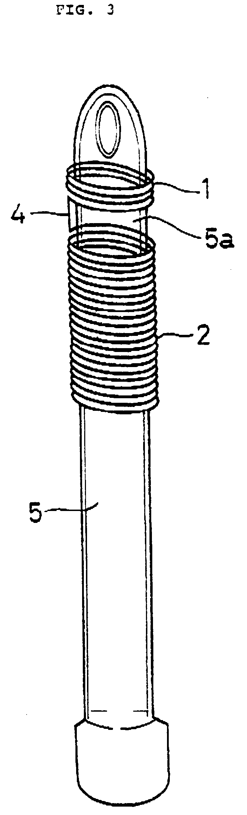

In preparation, as shown in Fig. 3, a tightly coiled SMA urethral stent is fitted around a Foley catheter 5 such that the proximal portion 1 of the stent is at the insertion tip side of the catheter 5 and interspace 3 of the SMA stent is at a bag portion or balloon portion 5a of the catheter 5.

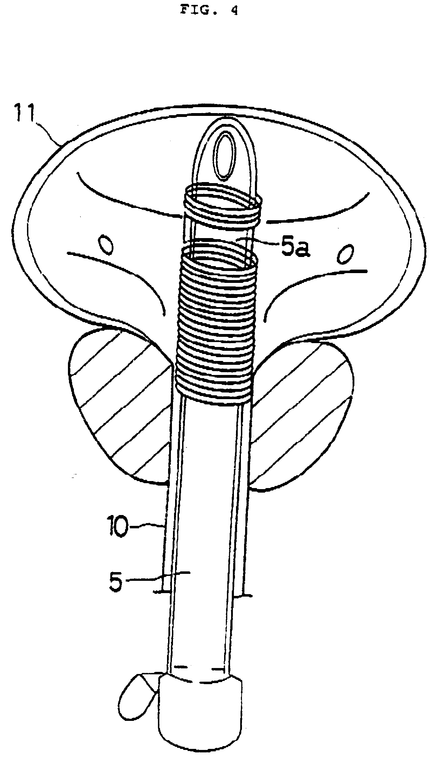

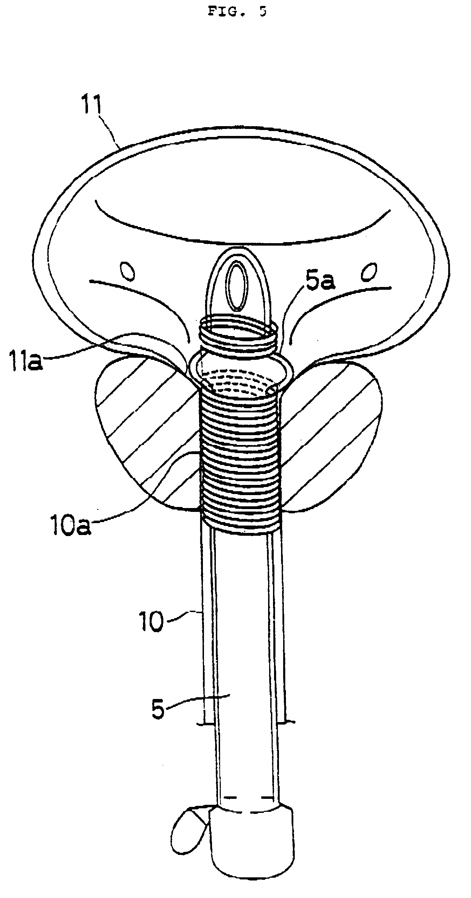

To locate the stent, firstly the Foley catheter 5 with the stent prepared as above is introduced into the urethra 10 and passed into the bladder cavity 11 in such a way that the proximal portion 1 and the interspace portion 3 corresponding to the bag portion 5a enter the bladder cavity 11, as shown in Fig. 4. By infusing water into the catheter 5, the bag portion 5a bulges out from the interspace 3 of the SMA stent. Accurate positioning of the SMA stent may be achieved by pulling the stent outwardly until the bulged bag portion 5a comes to abut against the bladder cervical region 11a, as shown in Fig. 5.

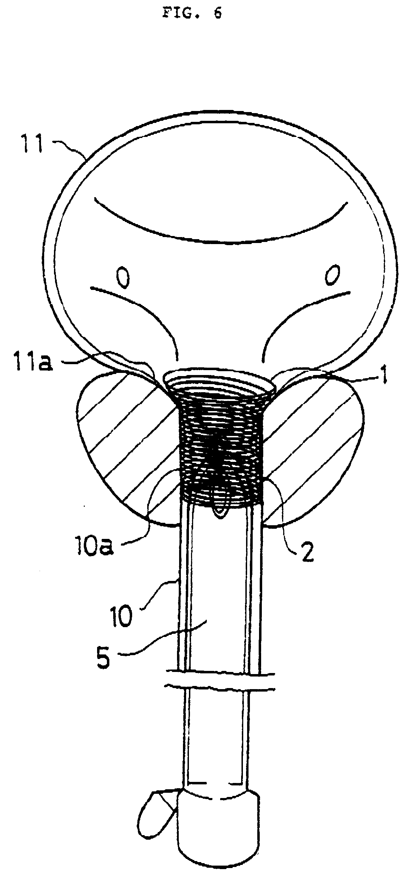

Subsequently the catheter 5 is deflated. After that, a flush of saline at a temperature of 41°C or above is passed through the catheter 5 to warm the SMA stent above the Af temperature. The stent, which changes shape due to thermally induced expansion as shown in Fig. 6, is positioned at the narrowing segment 11a, 10a of the urethra 10. Thus, the proximal portion 1 is located at the bladder cervical region 11a and the distal portion 2 is located in the urethra 10a at the prostate. At this stage removal of the Foley catheter 5 leaves the stent in situ and located in the desired portion. Since the stent is in the expanded state, the catheter 5 is easily removed from within the stent.

When the stent is no longer required or alternatively needs to be renewed, removal of the SMA stent is to be performed as follows.

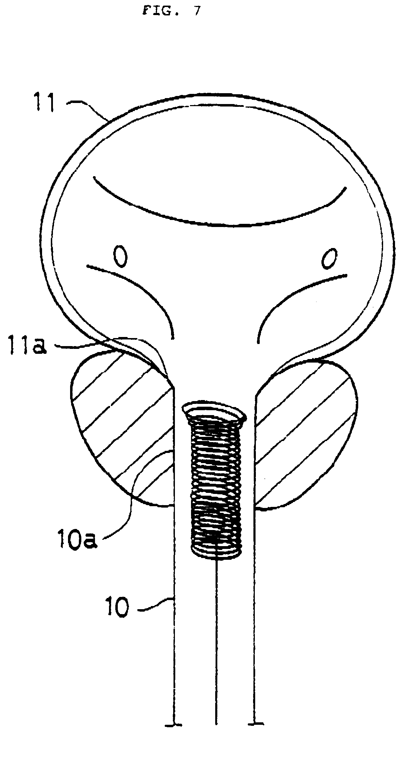

A Foley catheter is introduced into the retaining SMA stent. A flush of cold saline (below the Martensitic transforming temperature of 25°C) into the catheter cools the stent, which actuates super-elastic transformation of the stent so that the stent adopts the shape of a fine cylinder (as shown in Fig. 7) or a straightened wire, so that the stent may then be withdrawn from a body orifice. It is preferable that the stent has string attached at the end of the distal portion 2, so that the stent is readily removable by hand.

The present invention is not limited to a urethral stent, rather the stents may be used also for the biliary or vascular system. Stents for use with the biliary or vascular system are typically smaller in diameter, but the diameter of the entire passage should measure about 1mm or more to ensure a blood or bile flow without an encrustation or impact, so that the stent may be retained for a longer period.

For location of an SMA stent for biliary or vascular system, a guide wire and catheter is necessary. In preparation, an SMA stent is applied around the catheter 5 in the same way for the urethral stent.

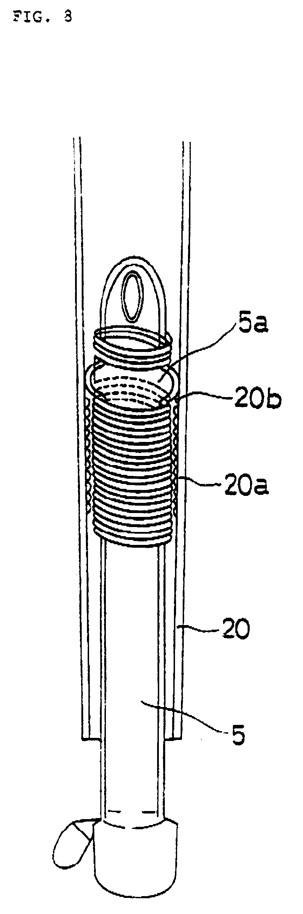

First a 0.35mm flexible tipped guide wire is inserted into a narrow segment 20a of the biliary tract or the blood vessel 20. Then the catheter which carries the SMA stent is passed over the guide wire so that the proximal portion 1 and the interspace portion 3 of the stent pass through a stricture 20a. Then in the same way as the above for the urethra stent, water is infused into the catheter, causing the bag portion 5a of the catheter 5 to bulge. The catheter 5 and stent are subsequently pulled outwardly until the stent abuts against the narrowing interior end wall 20b of the narrow segment 20a, as shown in Fig. 8. This presents the stent in the desired position.

After the positioning stage, the catheter 5 is deflated. Subsequently saline at a temperature of from 41°C to 43°C is infused through the catheter to heat it up to approximately the same temperature. This causes the stent to expand and loosen from the catheter 5, and furthermore to retain in contact with the entire wall of mucosa. Pulling the catheter 5 from the stent leaves the stent in the right position in the biliary tract or the blood vessel.

Removal of the SMA stent may be achieved by the same manner as the urethral stent. It is preferable that the stent has a floppy stainless steel tip at the end of the distal portion 2 to that the stent may be retrieved with a strong magnet device.

SMA stents for a biliary, urethral and vascular system according to embodiments of the present invention have the following advantages.

When a stent is inserted into the system, it is typically in the configuration of a contracted cylinder. This facilitates the insertion procedure. Also it allows the stent to expand to the desired shape at the Af temperature phase. The stent may be expanded in a simple manner by infusing saline at the Af temperature. Also the configuration at and above the Af temperature is preferably an expanded combination of a funnel portion and a cylindrical portion so that the funnel portion may contact with the narrowing interior end wall of the narrow segment and the stent is held in the right position. By cooling with cold saline, the stent is super-elastically transformed into a fine cylinder shape or a straightened wire, thereby allowing easy removal.

The stent of the present invention is more suitable for haemostatics after the bleeding relating to surgical intervention than any other manoeuvre such as traction with indwelled balloon catheter, since the expansion of the SMA stent allows the stent to compress directly towards the prostatic bed or the like. Indirect visceral wall compression through excessive traction at an area of bleeding inevitably causes adverse reactions.

## Claims

1. A stent suitable for use with a biliary, urinary or vascular system, the stent being made of a shape memory alloy having an Austenite finished temperature (Af) ranging from 41°C to 43°C, the stent having a first configuration at a temperature below the Af and a second expanded configuration at or above the Af.

2. A stent according to Claim 1 which comprises, in a first configuration, a distal cylindrical portion (2), a proximal cylindrical portion (1) and an interspace (3) therebetween and which is capable of adopting a second configuration in which the proximal portion (1) is flared away from the distal portion (2), the width of the proximal portion (1) is increased and the interspace (3) is reduced as compared with the first configuration.

3. A stent according to Claim 1 or 2 wherein the proximal portion (1) and the distal portion (2) are coiled.

4. A stent according to Claim 1 or 2 wherein the proximal portion (1) and the distal portion (2) are made of mesh.

5. A stent according to any preceding claim, wherein in a first configuration the cylindrical portions (1, 2) of the stent are of ellipsoidal or circular cross section.

6. A stent according to any preceding claim, wherein in a first configuration the length of the distal portion (2) is longer than that of the proximal portion (1).

7. A stent according to any preceding claim, wherein in a first configuration the two portions (1, 2) are connected by a straight wire portion (4).

8. A method of locating a stent as claimed in any one of the preceding claims in a desired position in a biliary, urinary or vascular system, which method comprises inserting the stent in a first configuration into a system and subsequently raising the temperature of the stent to or above its Af so that the stent assumes a second expanded configuration.

9. A method according to Claim 8 in which the temperature of the stent is raised by contacting the stent with fluid having a temperature at or above the Af.

10. A method according to Claim 9 which includes mounting a stent, which has in a first configuration a distal cylindrical portion, a proximal cylindrical portion and an interspace therebetween, on an inflatable catheter, inserting the catheter and stent into the system, inflating the catheter at least at or near the interspace of the stent so that the inflated catheter facilitates accurate positioning of the stent, and raising the temperature of the stent so that it assumes its second configuration.
